(19) 

Europäisches
Patentamt
European
Patent Office
Office européen
des brevets

(11)　**EP 3 438 653 A1**

(12)　**EUROPEAN PATENT APPLICATION**
published in accordance with Art. 153(4) EPC

(43) Date of publication:
　　 **06.02.2019　Bulletin 2019/06**

(21) Application number: **17775843.0**

(22) Date of filing: **30.03.2017**

(51) Int Cl.:
　　 **G01N 27/327** (2006.01)　　　**G01N 33/00** (2006.01)
　　 **G01N 25/32** (2006.01)

(86) International application number:
　　 **PCT/KR2017/003461**

(87) International publication number:
　　 **WO 2017/171419 (05.10.2017 Gazette 2017/40)**

(84) Designated Contracting States:
　　 **AL AT BE BG CH CY CZ DE DK EE ES FI FR GB
　　 GR HR HU IE IS IT LI LT LU LV MC MK MT NL NO
　　 PL PT RO RS SE SI SK SM TR**
　　 Designated Extension States:
　　 **BA ME**
　　 Designated Validation States:
　　 **MA MD**

(30) Priority: **30.03.2016　KR 20160038471**

(71) Applicant: **Cometnetwork Co., Ltd.
　　 Haeundae-gu, Busan 48059 (KR)**

(72) Inventor: **PARK, Jin Su
　　 Suwon-si
　　 Gyeonggi-do 16682 (KR)**

(74) Representative: **Conti, Marco
　　 Bugnion S.p.A.
　　 Via di Corticella, 87
　　 40128 Bologna (IT)**

(54)　**SENSOR FOR MEASURING CONCENTRATION OF NITROGEN OXIDE AND DETECTING AMMONIA SLIP**

(57)　The present invention relates to a gas sensor and, more specifically, to a gas sensor capable of measuring the concentration of nitrogen oxide and detecting ammonia slip. The present invention provides a sensor for measuring the concentration of nitrogen oxide and detecting ammonia slip, comprising: an oxygen ion-conductive solid electrolyte; a first electrode coming into contact with the solid electrolyte, and being reactive with nitrogen oxide; a second electrode coming into contact with the solid electrolyte, separated from the first electrode, and being reactive with nitrogen oxide; a third electrode coming into contact with the solid electrolyte, separated from the second electrode, connected in parallel with the first electrode, and being reactive with ammonia; a power supply device formed so as to apply power between the second electrode and the first electrode and third electrode, which are connected in parallel with each other; and a measurement device formed so as to measure the potential difference or the current between the first electrode and the third electrode, which are connected in parallel with each other, and the second electrode. According to the present invention, the sensor for measuring the concentration of nitrogen oxide and detecting ammonia slip can simultaneously measure nitrogen monoxide and nitrogen dioxide. In addition, whether ammonia is discharged can be simultaneously measured. According to the present invention, manufacturing costs of the sensor for measuring the concentration of nitrogen oxide and detecting ammonia slip are reduced since a nitrogen oxide sensor and an ammonia sensor share a part of the electrodes, the solid electrolyte, a heating part and the like. In addition, installation costs are also reduced since the nitrogen oxide sensor and the ammonia sensor do not need to be separately installed.

FIG.2

**Description**

FIELD OF THE INVENTION

The present invention relates to a gas sensor, and more particularly, the gas sensor capable of not only measuring concentration of nitrogen oxides but also detecting ammonia slip.

BACKGROUND OF THE INVENTION

Nitrogen oxides, as compounds of oxygen and nitrogen contained in air and fuels, contain nitrogen monoxide (NO), nitrogen dioxide ($NO_2$), dinitrogen trioxide ($N_2O_3$), and nitrous oxide ($N_2O$) and they are expressed as NOx. Among them, nitrogen monoxide and nitrogen dioxide are the most abundant nitrogen oxides, which work as air pollutants. Accordingly, it is necessary to properly control their emissions by measuring their concentration.

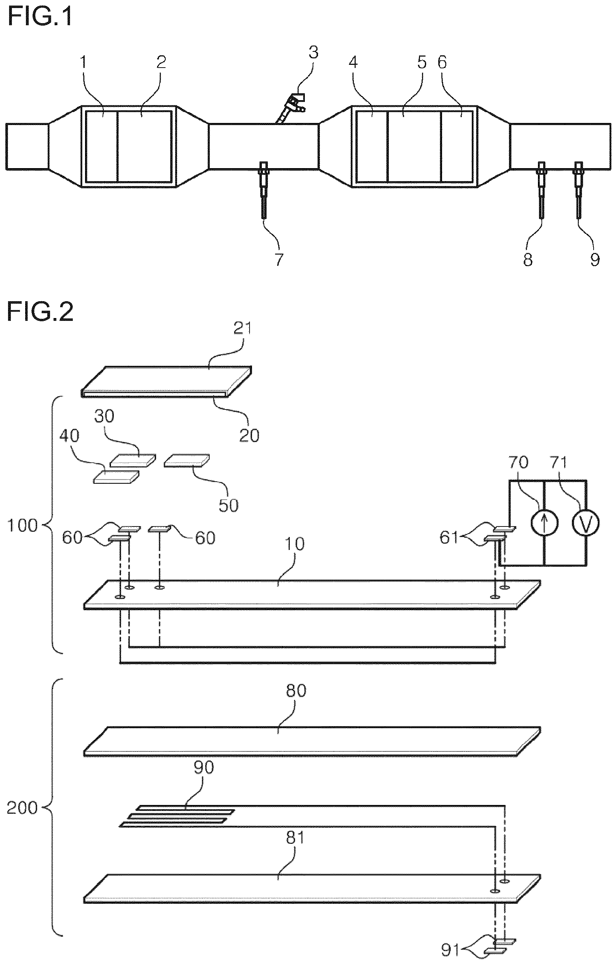

Internal combustion engines of vehicles, vessels, power plants, heavy equipment, agricultural machines and so forth are primary sources of emitting NOx. To remove NOx emitted from such internal combustion engines, a selective catalytic reduction (SCR) system illustrated in Fig. 1 is applied. As illustrated in Fig. 1, the SCR system includes a diesel oxidation catalyst 1, a smoke reducing apparatus 2, a urea solution injector 3, a hydrolysis unit 4, an SCR 5, and an ammonia oxidation catalyst 6 to remove harmful substances included in exhaust gas.

Furthermore, it includes a nitrogen oxide sensor 7 for controlling a system, and a nitrogen oxide sensor 8 and an ammonia sensor 9 installed in a rear end part for diagnosing faults. The nitrogen oxide sensor 7 in a front-end part measures concentration of nitrogen oxides emitted in real time and delivers it to the urea solution injector 3. The urea solution injector 3 injects a proper amount of urea solution to exhaust gas based on a measured value received from the sensor 7 for measuring nitrogen oxides emitted. NOx conversion efficiency is reduced when the urea solution is not fully injected, and ammonia is emitted into an atmosphere when it is too much injected. Amount of unreacted ammonia emitted from the SCR system to the atmosphere means "ammonia slip".

In the rear end part of the system, the nitrogen oxide sensor 8 measures concentration of nitrogen oxides emitted to check degradation or faults of the SCR system. The ammonia sensor 9 detects occurrence of the ammonia slip.

Patent Literature: KR 10-0864381

SUMMARY OF THE INVENTION

There has been a conventional problem of installing a nitrogen oxide sensor and an ammonia sensor separately in a rear-end part of a selective catalytic reduction (SCR) system to diagnose degradation and faults of the SCR system.

It is an object of the present invention to solve the problem, particularly, not only to precisely measure concentration of nitrogen oxides but also to provide a new gas sensor for detecting ammonia slip.

To achieve the object above, the present invention provides a sensor for measuring concentration of nitrogen oxides and detecting ammonia slip, comprising an oxygen ion conductive solid electrolyte; a first electrode, contacting the solid electrolyte, reactive to nitrogen oxides, a second electrode, contacting the solid electrolyte, reactive to nitrogen oxides while being separated from the first electrode, a third electrode, contacting the solid electrolyte, reactive to ammonia while being separated from the second electrode and being connected with the first electrode in parallel, a power supply configured to supply power between the first electrode and the third electrode connected with each other in parallel, and the second electrode, and a measuring instrument configured to measure electric potential difference or an electric current between the first electrode and the third electrode connected with each other in parallel, and the second electrode.

In addition, the present invention provides a sensor wherein the solid electrolyte is a plate type; and the first, the second, and the third electrodes are formed on one side of the solid electrolyte.

In addition, the present invention provides a sensor wherein the solid electrolyte is a plate type; the first electrode and the third electrode are formed on one side of the solid electrolyte and the second electrode is formed on the other side of the solid electrolyte.

In addition, the present invention provides a sensor further comprising a catalytic bed for oxidation of ammonia formed on a path where ammonia flows toward the third electrode.

In addition, the present invention provides a sensor wherein the catalytic bed for oxidation of ammonia is formed on a surface of the solid electrolyte.

In addition, the present invention provides a sensor wherein the catalytic bed for oxidation of ammonia includes at least one of substances selected from Pt, Pd, Rh, Ir, Ru, and Ag.

In addition, the present invention provides a sensor wherein the catalytic bed for oxidation of ammonia includes a porous ceramic containing precious metals scattered.

In addition, the present invention provides a sensor wherein the catalytic bed for oxidation of ammonia includes at least one of substances selected from $CO_3O_4$, $MnO_2$, $V_2O_5$, $Ni-Al_2O_3$, $Fe-Al_2O_3$, $Mn-Al_2O_3$, $CuO-Al_2O_3$, $Fe_2O_3-Al_2O_3$, $Fe_2O_3-TiO_2$, and $Fe_2O_3-ZrO_2$.

In addition, the present invention provides a sensor wherein the catalytic bed for oxidation of ammonia includes ion exchanging zeolite.

In addition, the present invention provides a sensor wherein the third electrode includes at least one of substances selected from $ZnO$, $SnO_2$, and $In_2O_3$.

In addition, the present invention provides a sensor for measuring concentration of nitrogen oxides and detecting ammonia slip, comprising an oxygen ion conductive solid electrolyte, a second electrode, contacting the solid electrolyte, reactive to nitrogen oxides, a fourth electrode including a first electrode layer, contacting the solid electrolyte, reactive to nitrogen oxides while being separated from the second electrode, and a third electrode layer reactive to ammonia, and a power supply configured to supply power between the second electrode and the fourth electrode, and a measuring instrument configured to measure electric potential difference or an electric current between the second electrode and the fourth electrode.

In addition, the present invention provides a sensor wherein the solid electrolyte is a plate type; and the second electrode and the fourth electrode are formed on one side of the solid electrolyte.

In addition, the present invention provides a sensor wherein the solid electrolyte is a plate type; and the second electrode is formed on one side of the solid electrolyte and the fourth electrode is formed on the other side thereof.

In addition, the present invention provides a sensor further comprising a catalytic bed for oxidation of ammonia formed on a path where ammonia flows toward the fourth electrode.

In addition, the present invention provides a sensor wherein the catalytic bed for oxidation of ammonia is formed on a surface of the solid electrolyte.

In addition, the present invention provides a sensor for measuring concentration of nitrogen oxides and detecting ammonia slip, comprising an oxygen ion conductive solid electrolyte, a second electrode, contacting the solid electrolyte, reactive to nitrogen oxides, a fifth electrode including a first electrode material, contacting the solid electrolyte, reactive to nitrogen oxides while being separated from the second electrode, and the third electrode material reactive to ammonia, a power supply configured to supply power between the second electrode and the fifth electrode, and a measuring instrument configure to measure electric potential difference or an electric current between the second electrode and the fifth electrode.

In addition, the present invention provides a sensor wherein the solid electrolyte is a plate type; and the second electrode and the fifth electrode are formed on one side of the solid electrolyte.

In addition, the present invention provides a sensor wherein the solid electrolyte is a plate type; and the second electrode is formed on one side of the solid electrolyte and the fifth electrode is formed on the other side of the solid electrolyte.

In addition, the present invention provides a sensor further comprising a catalytic bed for oxidation of ammonia formed on a path where ammonia flows towards the fifth electrode.

In addition, the present invention provides a sensor wherein the catalytic bed for oxidation of ammonia is formed on a surface of the solid electrolyte.

In addition, the present invention provides a sensor further comprising a plate-type supporter contacting the solid electrolyte, wherein the solid electrolyte is a plate type, and the supporter is placed on one or the other side of the solid electrolyte.

In addition, the present invention provides a sensor wherein the solid electrolyte is porous.

BRIEF DESCRIPTION OF THE DRAWINGS

Fig. 1 is a drawing illustrating a conventional selective catalytic reduction system.

Fig. 2 is an exploded perspective drawing of an example embodiment of a sensor for measuring concentration of nitrogen oxides and detecting ammonia slip in accordance with the present invention.

Fig. 3 is a perspective drawing of a measuring cell in an example embodiment of the sensor for measuring concentration of nitrogen oxides and detecting ammonia slip in accordance with the present invention.

Fig. 4 is graphs illustrating results of measuring voltages according to changes in concentration of nitrogen oxides and ammonia when a current source of $-5\mu A$ was used as a power supply of the sensor for measuring concentration of nitrogen oxides and detecting ammonia slip in Fig. 2.

Fig. 5 is a perspective drawing of a measuring cell in an example embodiment of the sensor for measuring concentration of nitrogen oxides and detecting ammonia slip in accordance with the present invention.

Fig. 6 is a perspective drawing of a measuring cell in an example embodiment of the sensor for measuring concentration of nitrogen oxides and detecting ammonia slip in accordance with the present invention.

Fig. 7 is a perspective drawing of a measuring cell in an example embodiment of the sensor for measuring concentration of nitrogen oxides and detecting ammonia slip in accordance with the present invention.

Fig. 8 is a perspective drawing of a measuring cell in an example embodiment of the sensor for measuring concentration of nitrogen oxides and detecting ammonia slip in accordance with the present invention.

Fig. 9 is a perspective drawing of a measuring cell in an example embodiment of the sensor for measuring concentration of nitrogen oxides and detecting ammonia slip in accordance with the present invention.

Fig. 10 is an exploded perspective drawing of a measuring cell in an example embodiment of the sensor for measuring concentration of nitrogen oxides and detecting ammonia slip in accordance with the present invention.

Fig. 11 is an exploded perspective drawing of a measuring cell in an example embodiment of the sensor for measuring concentration of nitrogen oxides and detecting ammonia slip in accordance with the present invention.

## DETAILED DESCRIPTION OF THE PREFERRED EMBODIMENTS

Detailed explanation of desirable example embodiments of the present invention is made by referring to attached drawings. Features of benefits of the present invention and a method for achieving them could be clarified by referring to example embodiments to be explained below with drawings attached. However, the present invention may not be limited to the example embodiments published herein but be implemented in a variety of different forms. The example embodiments of the present invention are provided to make the present invention perfect and to perfectly inform those skilled in the art of the category of the present invention. The present invention is defined only by the category of claims. The same reference numbers over the whole specification indicate the same components.

Unless there is otherwise any definition, all terms used in this specification (including technical and scientific terms) could be used in meanings commonly understood by those skilled in the art in the technical field to which the present invention belongs. Unless clearly and specially defined, terms defined in dictionaries generally used are not abnormally or excessively interpreted.

The terms used in this specification are used to explain example embodiments but not to limit the present invention. In this specification, unless otherwise specifically stated in a phrase, a singular term shall include even plural ones. The words such as "comprises," and/or "comprising" used in this specification shall not exclude existence or addition of other components other than the stated component.

Fig. 2 is an exploded perspective drawing of an example embodiment of a sensor for measuring concentration of nitrogen oxides and detecting ammonia slip in accordance with example embodiment of the present invention. By referring to Fig. 2, the example embodiment of the sensor for measuring concentration of nitrogen oxides and detecting ammonia slip in accordance with the example embodiment of the present invention comprises a measuring cell 100 and a heating part 200 for heating the measuring cell 100 up to a temperature where the measuring cell 100 may work.

The measuring cell 100 includes: a first supporting layer 10; an oxygen ion conductive solid electrolyte 20 layered on the first supporting layer 10; a first, a second, and a third electrode 30, 40, and 50, which are placed between the solid electrolyte 20 and the first supporting layer 10 and contact the solid electrolyte 20; current collectors 60 formed on the electrodes 30, 40, and 50. Besides, it includes terminals 61 connected to the current collectors 60; and a power supply 70 and a measuring instrument 71 which are connected to the terminals 61.

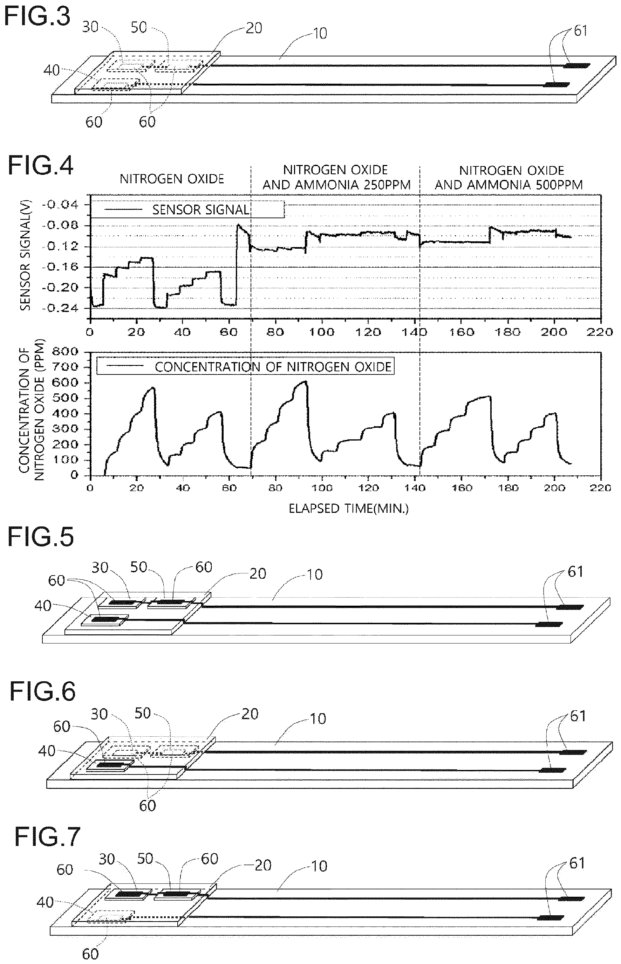

Fig. 3 is a perspective drawing of a measuring cell 100. In Fig. 3, a first supporting layer 10, a solid electrolyte 20, electrodes 30, 40, and 50, current collectors 60 and terminals 61, which form the measuring cell 100, are indicated and the other components of the measuring cell 100 are omitted. On one side of the plate-type solid electrolyte 20, the first, the second, and the third electrodes 30, 40, and 50 are formed. Besides, the first supporting layer 10, as a plate-type supporter that supports the solid electrolyte 20, is equipped on the one side of the solid electrolyte 20. In other words, the first, the second, and the third electrodes 30, 40, and 50 being placed between the one side of the solid electrolyte 20 and the first supporting layer 10 are equipped on the one side of the solid electrolyte 20.

Moreover, in Fig. 2, lead wires connecting the current collectors 60 with the terminals 61 are formed on the one side of the first supporting layer 10 but in Fig. 3, lead wires connecting the current collectors 60 with the terminals 61 are formed on the other side of the first supporting layer 10. In the present invention, the lead wires connecting the current collectors 60 and the terminals 61 may be formed either on one side of the first supporting layer 10 or on the other side thereof.

In Figs. 2 and 3, it is illustrated that all the first, the second, and the third electrodes 30, 40, and 50 are formed on the same side, but some of them may be formed on the other side thereof. If the solid electrolyte 20 is placed between positive electrodes, e.g., the first and the third electrodes, and a negative electrode, e.g., the second electrode, oxygen ions move in thickness direction of the solid electrolyte 20, and if the positive and negative electrodes are placed on the same side, the oxygen ions move generally in orthogonal direction to the thickness direction through the solid electrolyte 20 between the positive and negative electrodes.

The oxygen ion conductive solid electrolyte 20, which is capable of conducting oxygen ions at a high temperature, may include stabilized zirconia, $CeO_2$ or $ThO_2$, etc. In addition, the solid electrolyte 20 is porous and contacts a porous catalytic bed 21 for oxidation of ammonia. Additionally, penetrating pores, through which gases are passed to the one side contacting the first supporting layer 10 from the other side thereof opposite to the one side, are formed. From these, the measuring cell 100 is configured to allow the gases which are to be measured to reach the first, the second, and the third electrodes 30, 40, and 50 by penetrating the inside of the porous catalytic bed 21 for oxidation of ammonia and the inside of the solid electrolyte 20 from the outside of the porous catalytic bed 21 for oxidation of ammonia.

Furthermore, the solid electrolyte 20 may be dense. If the solid electrolyte 20 is to be made dense, a flow path through which the gases pass from one side of the solid electrolyte 20 to the other side thereof, for example, may be formed in the solid electrolyte 20.

The first electrode 30 and the second electrode 40 may be metal oxide semiconductor materials reactive to nitrogen oxides and oxygen when power is supplied to them. The first electrode 30 and the second electrode 40 may be metal oxide semiconductor materials which are same as, or different from, each other. For instance, the first electrode 30 and the second electrode 40 may include at least one of substances selected from a group composed of $CuO$, $NiO$, $CoO$, $Cr_2O_3$, $Cu_2O$, $MoO_2$, $Ag_2O$, $Bi_2O_3$, $Pr_2O_3$, $ZnO$, $MgO$, $V_2O_5$, $Fe_2O_3$, $TiO_2$, $CeO_2$, $WO_3$ and $MnO$.

To make concentration of the nitrogen oxides and an electrical signal produced from the measuring instrument have more delicately proportional relationships, the first electrode 30 and the second electrode 40 may also include solid electrolytes, non-conductive oxide, glass, and precious metals.

The third electrode 50 may be a metal oxide semiconductor material reactive to ammonia and oxygen when power is supplied to it. For example, the third electrode 50 may include at least one of substances selected from a group composed of $ZnO$, $SnO_2$, and $In_2O_3$. The third electrode 50 and the first electrode 30 are connected with each other in parallel. In other words, one surface of the first electrode 30 and that of the third electrode 50 contact the solid electrolyte 20 and the other surfaces of them, which do not contact the solid electrolyte 20, are electronically connected with each other.

Moreover, the sensor for measuring concentration of nitrogen oxides and detecting ammonia slip in accordance with the example embodiment comprises the catalytic bed 21 for oxidation of ammonia formed in the path where ammonia flows through the third electrode 50. For example, as illustrated in Fig. 2, it may form the catalytic bed 21 for oxidation of ammonia on a surface of the solid electrolyte 20 to make only ammonia gas with concentration exceeding specific concentration reach the third electrode 50. The catalytic bed 21 for oxidation of ammonia may include at least one substance selected from precious metals such as Pt, Pd, Rh, Ir, Ru, and Ag, porous ceramics containing precious metals scattered, mixed metal oxide catalysts such as $CO_3O_4$, $MnO_2$, $V_2O_5$, $Ni\text{-}Al_2O_3$, $Fe\text{-}Al_2O_3$, $Mn\text{-}Al_2O_3$, $CuO\text{-}Al_2O_3$, $Fe_2O_3\text{-}Al_2O_3$, $Fe_2O_3\text{-}TiO_2$, and $Fe_2O_3\text{-}ZrO_2$, ion exchanging zeolite and so forth.

The catalytic bed 21 for oxidation of ammonia plays a role in oxidizing and removing the ammonia gas. Accordingly, the concentration of the ammonia gas that may reach the third electrode 50 may be adjusted by adjusting thickness of the catalytic bed 21 for oxidation of ammonia.

The first, the second, and the third electrodes 30, 40, and 50 are individually combined with the current collectors 60. It is desirable that the current collectors 60 may be made as electrically conductive metals, or as precious metals to withstand in a corrosion environment. As a precious metal, at least one of gold (Au), silver (Ag), platinum (Pt), iridium (Ir), palladium (Pd) or substances selected from such alloys may be applied and it is desirable to use gold or platinum.

The power supply 70 supplies power between the first electrode 30 and the third electrode 50 connected with each other in parallel, and the second electrode 40 through the terminals 61 connected with the current collectors 60. In Fig. 2, it is illustrated that the power supply 70 is a current source but the power supply 70 could be a voltage source.

The measuring instrument 71 measures electric potential difference or an electric current between the first electrode 30 and the third electrode 50 connected with each other in parallel, and the second electrode 40 through the terminals 61 connected with the current collectors 60. If a current source is used as the power supply 70, the measuring instrument 71 could be a voltmeter and if a voltage source is used as the power supply 70, the measuring instrument 71 could be an ammeter.

By referring to Fig. 2, the first electrode 30 and the third electrode 50 may be defined as positive electrodes and the second electrode 40 as a negative electrode, or vice versa.

Below explanation will be made on principles of measuring concentration of nitrogen oxides and detecting ammonia on assumption that the first electrode 30 and the third electrode 50 are positive electrodes, for convenience.

There occurs an anodic reaction, through which oxygen ions are converted into oxygen gas in an interface between the first electrode 30 as a positive electrode and the solid electrolyte 20, and at the same time, if there is nitrogen monoxide (NO) gas, the anodic reaction occurs by the nitrogen monoxide as shown in Chemical Formula 1. If a current source is used as a power supply, a voltage between the first electrode 30 and the second electrode 40 is reduced to pass a certain electric current, and if a voltage source is a used as a power supply, a size of the electric current being passed between the first electrode 30 and the second electrode 40 increases to keep a certain voltage.

[Chemical Formula 1]

$$O^{2-} = \frac{1}{2}O_2 + 2e^-, \quad NO + O^{2-} = NO_2 + 2e^-$$

There occurs a cathodic reaction, through which oxygen gas is converted into oxygen ions in an interface between the second electrode 40 as a negative electrode and the solid electrolyte 20, and at the same time, if there is nitrogen dioxide ($NO_2$) gas, the anodic reaction occurring by the nitrogen dioxide as shown in Chemical Formula 2 increases size of a voltage to pass a certain electric current and increases the electric current to keep a certain voltage.

[Chemical Formula 2]

$$\frac{1}{2}O_2 + 2e^- = O^{2-}, \quad NO_2 + 2e^- = NO + O^{2-}$$

As such, if the first electrode 30 and the second electrode 40 of the sensor illustrated in Fig. 2 are exposed to gases mixed with nitrogen oxides, the sensor may measure total concentration of nitrogen dioxide and nitrogen monoxide as a size of an electric signal changes such as electric potential difference or a size of the electric current according to concentration of nitrogen dioxide and nitrogen monoxide in the nitrogen oxide gas.

Moreover, there occurs an anodic reaction through which oxygen ions are converted into oxygen gas in an interface between the third electrode 50 electrically connected with the first electrode 30 as a positive electrode and the solid electrolyte 20, and at the same time, if there is ammonia ($NH_3$) gas, the anodic reaction occurring by the ammonia as shown in Chemical Formula 3 reduces size of a voltage to pass a certain electric current and increases the electric current to keep a certain voltage.

[Chemical Formula 3]

$$O^{2-} = \frac{1}{2}O_2 + 2e^-, \quad 2NH_3 + 3O^{2-} = N_2 + 3H_2O + 6e^-$$

Fig. 4 is graphs illustrating a result of measuring voltages according to changes in concentration of nitrogen oxides and ammonia when a current source of -5$\mu$A was used as a power supply of the sensor for measuring concentration of nitrogen oxides and detecting ammonia slip in Fig. 2. As a first electrode 30, NiO was used while $LaCoO_3$ was used as a second electrode 40 and $In_2O_3$ as a third electrode 50 and stabilized zirconia as a conductive solid electrolyte 20. Platinum was used as the current collectors 60. Voltages were measured while concentration of nitrogen oxides and ammonia was being changed at a 20% oxygen tension.

As illustrated in Fig. 4, the sensor for measuring concentration of nitrogen oxides and detecting ammonia slip produces a different voltage value depending on high or low concentration of ammonia.

When the concentration of ammonia is low, it can be found out that the sensor for measuring concentration of nitrogen oxides and detecting ammonia slip produces a voltage value in proportion to the whole concentration of nitrogen oxide. In other words, the sensor for measuring concentration of nitrogen oxides and detecting ammonia slip produces a value between -240mV and -140mV in proportion to the concentration of nitrogen oxides in this section. The ammonia included in the exhaust gas is ignored. At the time, a concentration value of ammonia that could be ignored may be determined by thickness and composition of a catalytic bed 21 for oxidation of ammonia surrounding the solid electrolyte 20. For example, only if ammonia slip is determined on condition that the concentration of ammonia is at least 50ppm or higher, ammonia with concentration of less than 50ppm is removed before it reaches the third electrode 50 and thickness of the catalytic bed 21 for oxidation of ammonia is adjusted to make only ammonia with concentration of more than 50ppm reach to the third electrode 50.

If the concentration of ammonia is at least 250ppm or higher, the sensor for measuring concentration of nitrogen oxides and detecting ammonia slip produces a value between - 120mV and -80mV by anodic reaction at the third electrode 50, regardless of concentration of nitrogen oxide.

In brief, the sensor for measuring concentration of nitrogen oxides and detecting ammonia slip in accordance with the present invention may accurately measure the concentration of nitrogen oxides within the scope of concentration of ammonia being not determined as ammonia slip because concentration of ammonia in an exhaust gas is low and may detect ammonia slip within the scope of concentration of ammonia being determined as ammonia slip.

Again, by referring to Fig. 2, the heating part 200 is explained below. The heating part 200 includes a second supporting layer 80, a third supporting layer 81, and a heater 90 placed between them. The heater 90 is in a serpentine form to increase a heating value. Not being illustrated, an insulating layer could be also formed on the upper side and the bottom side of the heater 90. The insulating layer may be composed of a ceramic whose main ingredient is alumina ($Al_2O_3$). The heating part 200, for example, may be manufactured in a method of printing a heater made of platinum, etc. on a green sheet of a third supporting layer, laminating a green sheet of a second supporting layer on the heater, compressing the green sheet of the second supporting layer and the green sheet of the third supporting layer, and heat-treating the compressed combination of the green sheet of the second supporting layer and the heater-printed green sheet of the third supporting layer.

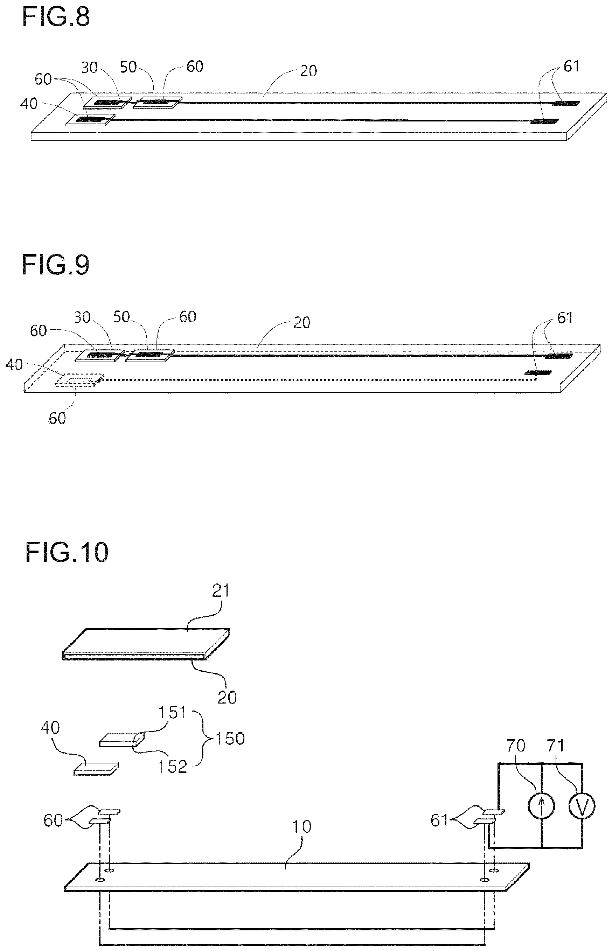

Figs. 5 to 11 illustrate perspective drawings of the measuring cells 100 in other example embodiments of the sensor for measuring concentration of nitrogen oxides and detecting ammonia slip in accordance with the present invention. In the example embodiments illustrated in Figs. 5 to 7, only the first supporting layer 10, the solid electrolyte 20, and electrodes 30, 40, and 50, the current collectors 60 and the terminals 61 which are comprised by the measuring cell 100 are presented and the other components of the measuring cell 100 are omitted. In the example embodiments illustrated in Figs. 8 and 9, only the solid electrolyte 20, electrodes 30, 40, and 50, the current collectors 60, and the terminals 61, which are comprised by the measuring cell 100, are additionally presented and the other components of the measuring cell 100 are omitted.

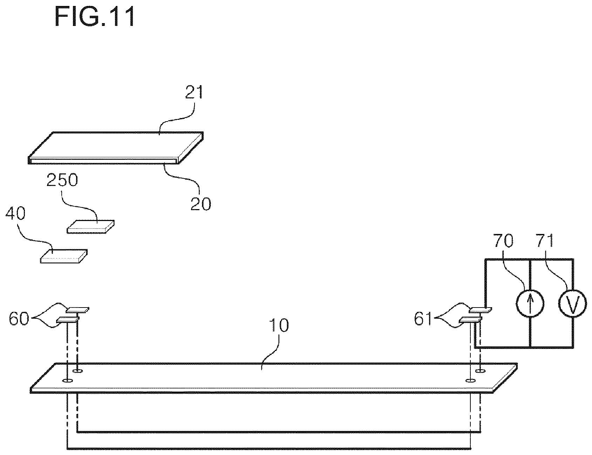

Besides, in Figs. 5 to 7, lead wires connecting the current collectors 60 with the terminals 61 are formed on a surface of the first supporting layer 10 where electrodes are formed. In Figs. 8 and 9, lead wires connecting the current collectors 60 with the terminals 61 are formed on one surface of the solid electrolyte 20 where electrodes are formed. In Figs. 10 and 11, lead wires connecting the current collectors 60 with the terminals 61 are formed on one surface of the first supporting layer 10 opposite to the other side thereof where electrodes are formed. As such, the lead wires connecting the current collectors 60 with the terminals 61 in the present invention may be formed on one side of the first supporting layer 10 or on one side of the solid electrolyte 20 or also on the other side thereof.

The example embodiment illustrated in Fig. 5 is different from those in Figs. 2 and 3 from the aspect that the first supporting layer placed on the one side of the solid electrolyte 20 in Figs. 2 and 3 is placed on the other side thereof in Fig. 5. Moreover, it has been explained that the upper side of the solid electrolyte 20 in Figs. 2 and 3 is indicated as the other side thereof and the bottom side thereof as one side but the upper side the solid electrolyte 20 in Fig. 5 is indicated as one side thereof and the bottom thereof is as the other side thereof.

More specifically, as illustrated in Fig. 5, the first supporting layer 10 is equipped on the other side of the plate-type solid electrolyte 20. On one side of the solid electrolyte 20, the first, the second, and the third electrodes 30, 40, and 50 are formed. Besides, not being illustrated, the catalytic bed 21 for oxidation of ammonia is formed on the one side of the solid electrolyte 20 (See Fig. 2). In other words, the first, the second, and the third electrodes 30, 40, and 50, being placed between the one side of the solid electrolyte 20 and the catalytic bed 21 for oxidation of ammonia, are equipped on the one side of the solid electrolyte 20.

The example embodiment illustrated in Fig. 6 is different from those in Figs. 2 and 3 from the aspect that the second electrode 40, being placed on one side of the solid electrolyte 20 in Figs. 2 and 3, is formed on other side thereof in Fig. 6.

More specifically, as illustrated in Fig. 6, the first electrode 30 and the third electrode 50 are formed on the other side of the plate-type solid electrolyte 20. In addition, the second electrode 40 is formed on the other side of the solid electrolyte 20. Furthermore, not being illustrated, the catalytic bed 21 for oxidation of ammonia is formed on the other side of the solid electrolyte 20 (See Fig. 2). In brief, the first electrode 30 and the third electrode 50, which are placed between the one side of the solid electrolyte 20 and the first supporting layer 10, are equipped on the one side of the solid electrolyte 20. Besides, the second electrode 40, which is placed between the other side of the solid electrolyte 20 and the catalytic bed 21 for oxidation of ammonia, is equipped on the other side of the solid electrolyte 20.

The example embodiment illustrated in Fig. 7 is different from that in Fig. 6 from the aspect that the first supporting layer 10, being formed on the one side of the solid electrolyte 20 in Fig. 6, is formed on the other side thereof in Fig. 7. In Fig. 6, the upper side of the solid electrolyte 20 means the other side thereof, and the bottom side thereof means the one side thereof but in Fig. 7, the upper side thereof means the one side thereof and the bottom side thereof means the other side thereof.

More specifically, as illustrated in Fig. 7, the second electrode 40 is formed on the other side of the plate-type solid electrolyte 20. Furthermore, the first supporting layer 10 is equipped on the other side of the solid electrolyte 20. Besides, the first electrode 30 and the third electrode 50 are formed on the one side of the solid electrolyte 20. Moreover, not being illustrated, the catalytic bed 21 for oxidation of ammonia is formed on the one side of the solid electrolyte 20 (See Fig. 2). In short, the second electrode 40, which is placed between the other side of the solid electrolyte 20 and the first supporting layer 10, is equipped on the solid electrolyte 20. In addition, the first electrode 30 and the third electrode 50, which are placed between the one side of the solid electrolyte 20 and the catalytic bed 21 for oxidation of ammonia, are equipped on the one side of the solid electrolyte 20.

From the aspect that the measuring cell 100 in Fig. 8 does not have the first supporting layer 10 which plays a role as a plate-type supporter that supports the solid electrolyte 20, the example embodiment illustrated in Fig. 8 is different from that in Fig. 5. In this case, it can be configured, for example, that the solid electrolyte 20 has strength as much as the first supporting layer 10.

More concretely, as illustrated in Fig. 8, the first, the second, and the third electrodes 30, 40, and 50 are formed on the one side of the plate-type solid electrolyte 20. Besides, not being illustrated, the catalytic bed 21 for oxidation of ammonia is formed on the one side of the solid electrolyte 20 where the first, the second, and the third electrodes 30, 40, and 50 are formed (See Fig. 2). Furthermore, not being illustrated, the heating part 200 for heating the measuring cell 100 up to a temperature at which the measuring cell 100 works is installed on the other side of the solid electrolyte 20 (See Fig. 2). In short, the first electrode 30 and the third electrode 50, which are placed on the one side of the solid electrolyte 20 and the catalytic bed 21 for oxidation of ammonia, are equipped on the one side of the solid electrolyte 20.

From the aspect that the measuring cell 100 does not have the first supporting layer 10 which plays a role as a plate-type supporter that supports the solid electrolyte 20, the example embodiment illustrated in Fig. 9 is different from that in Fig. 7. In this case, it can be configured, for example, that the solid electrolyte 20 has strength as much as the first supporting layer 10.

Specifically, as illustrated in Fig. 9, the second electrode 40 is formed on the other side of the solid electrolyte 20. Not being illustrated, the heating part 200 for heating the measuring cell 100 up to a temperature at which the measuring cell 100 works is installed on the other side of the solid electrolyte 20. Furthermore, the first electrode 30 and the third electrode 50 are formed on the one side of the solid electrolyte 20. Not being illustrated, the catalytic bed 21 for oxidation of ammonia, moreover, is formed on the one side of the solid electrolyte 20 (See Fig. 2). In brief, the second electrode 40, which is placed between the other side of the solid electrolyte 20 and the heating part 200, is equipped on the other side of the solid electrolyte 20. Additionally, the first electrode 30 and the third electrode 50, which are placed between the one side of the solid electrolyte 20 and the catalytic bed 21 for oxidation of ammonia, are equipped on the one side of the solid electrolyte 20.

Fig. 10 is an exploded perspective drawing of a measuring cell in an example embodiment of the sensor for measuring concentration of nitrogen oxides and detecting ammonia slip. Because this example embodiment is different from that illustrated in Fig. 2 from the aspect that a fourth electrode 150 is used, instead of the first electrode 30 and the third electrode 50 in the example embodiment illustrated in Fig. 2, detailed explanation will be made only for this.

In this example embodiment, the second electrode 40 and the fourth electrode 150 are formed on one side of the plate-type solid electrolyte 20. Besides, the first supporting layer 10 is equipped on the one side of the solid electrolyte 20. Furthermore, the catalytic bed 21 for oxidation of ammonia is formed on the other side of the solid electrolyte 20. In short, the second electrode 40 and the fourth electrode 150, which are placed between the one side of the solid electrolyte 20 and the first supporting layer 10, are equipped on the one side of the solid electrolyte 20.

The fourth electrode 150 includes a first electrode layer 151 reactive to nitrogen oxides and a third electrode layer 152 reactive to ammonia. It is not specially limited, but the first electrode layer 151 may perform a role as an ammonia oxidation catalyst. Therefore, it is desirable to form the first electrode layer 151 on the solid electrolyte 20, and the third electrode layer 152 on the first electrode layer 151.

Just like the first electrode 30, the first electrode layer 151 may include at least one substance selected from a group composed of $CuO$, $NiO$, $CoO$, $Cr_2O_3$, $Cu_2O$, $MoO_2$, $Ag_2O$, $Bi_2O_3$, $Pr_2O_3$, $ZnO$, $MgO$, $V_2O_5$, $Fe_2O_3$, $TiO_2$, $CeO_2$, $WO_3$, and $MnO$.

Just like the third electrode 50, the third electrode layer 152 may include at least one of substances selected from a group composed of $ZnO$, $SnO_2$, and $In_2O_3$.

Besides, the catalytic bed 21 for oxidation of ammonia surrounding the solid electrolyte 20 maybe formed on a surface of the fourth electrode 150 to make only ammonia gas with at least a certain concentration reach.

The fourth electrode 150, for example, may be formed in a heat-treating method after a second electrode paste, a first electrode paste, and an ammonia oxidation catalyst paste in order in a screen-printing method on a green sheet of a solid electrolyte.

In the example embodiment illustrated in Fig. 10, the second electrode 40 and the fourth electrode 150 are formed on the one side of the solid electrolyte 20, but without being limited to this, one of the second electrode 40 and the fourth electrode 150 may be formed on the one side of the solid electrolyte 20 and the other may be formed on the other side thereof. In this case, the second electrode 40 or the fourth electrode 150 formed on the other side of the solid electrolyte 20, which are placed between the other side of the solid electrolyte 20 and the catalytic bed 21 for oxidation of ammonia, is equipped on the other side of the solid electrolyte 20.

Furthermore, in the example embodiment illustrated in Fig. 10, the first supporting layer 10 are equipped on the one side of the solid electrolyte 20 and the catalytic bed 21 for oxidation of ammonia is installed on the other side of the solid electrolyte 20 but without being limited to this, a first supporting layer 10 may be installed on the other side of the solid electrolyte 20 and the catalytic bed 21 for oxidation of ammonia may be installed on the one side thereof. In the case, the second electrode 40 and the fourth electrode 150, which are placed between the one side of the solid electrolyte 20 and the catalytic bed 21 for oxidation of ammonia, are equipped on the one side of the solid electrolyte 20.

Fig. 11 is an exploded perspective drawing of a measuring cell in an example embodiment of the sensor for measuring concentration of nitrogen oxides and detecting ammonia slip in accordance with the present invention. From the aspect that this example embodiment uses a fifth electrode 250, instead of the first electrode 30 and the third electrode 50 in the example embodiment illustrated in Fig. 2, this is different from that illustrated in Fig. 2. Therefore, only the difference will be explained in detail.

In this example embodiment, the second electrode 40 and the fifth electrode 250 are formed on the one side of the plate-type solid electrolyte 20. Furthermore, the first supporting layer 10 is equipped on the one side of the solid electrolyte 20. Moreover, the catalytic bed 21 for oxidation of ammonia is formed on the other side of the solid electrolyte 20. In brief, the second electrode 40 and the fifth electrode 250, which are placed between the one side of the solid electrolyte 20 and the first supporting layer 10, are equipped on the one side of the solid electrolyte 20.

The fifth electrode 250 includes a first electrode material reactive to nitrogen oxides and a third electrode material reactive to ammonia.

Just like the first electrode 30, the first electrode material may include at least one of substances selected from a group composed of $CuO$, $NiO$, $CoO$, $Cr_2O_3$, $Cu_2O$, $MoO_2$, $Ag_2O$, $Bi_2O_3$, $Pr_2O_3$, $ZnO$, $MgO$, $V_2O_5$, $Fe_2O_3$, $TiO_2$, $CeO_2$, $WO_3$ and $MnO$.

Just like the third electrode 50, the third electrode material may include at least one of substances selected from a group composed of $ZnO$, $SnO_2$, and $In_2O_3$.

The fifth electrode 250, for example, may be formed in a heat-treating method after a paste, which is manufactured by mixing the first electrode material, the third electrode material, a binder, a solvent, etc., is printed on a green sheet of a solid electrolyte.

Besides, the catalytic bed 21 for oxidation of ammonia may be formed on a surface of the solid electrolyte 20.

In the example embodiment illustrated in Fig. 11, the second electrode 40 and the fifth electrode 250 are formed on the one side of the solid electrolyte 20 but without being limited to these, either of the second electrode 40 and the fifth electrode 250 may be formed on the one side of the solid electrolyte 20 and the other on the other side of the solid electrolyte 20. In this case, the second electrode 40 or the fifth electrode 250, which are placed between the other side of the solid electrolyte 20 and the catalytic bed 21 for oxidation of ammonia, is equipped on the other side of the solid electrolyte 20.

Furthermore, in the example embodiment illustrated in Fig. 11, the first supporting layer 10 is equipped in the one side of the solid electrolyte 20 and the catalytic bed 21 for oxidation of ammonia is installed on the other side of the solid electrolyte 20, but without being limited to these, the first supporting layer 10 may be equipped on the other side of the solid electrolyte 20 and the catalytic bed 21 for oxidation of ammonia may be installed on the one side of the solid electrolyte 20. In this case, the second electrode 40 and the fifth electrode 250, which are placed between the one side of the solid electrolyte 20 and the catalytic bed 21 for oxidation of ammonia, are equipped in the one side of the solid electrolyte 20.

The desirable example embodiments of the present invention have been illustrated and explained above, but the present invention is not limited to the aforementioned specific example embodiments. Of course, it is possible for those skilled in the art to implement various alternations without being beyond the summary of the present invention claimed and it would not be understood that such alternations are separated from the technical spirit or prospect of the present invention.

For example, it is illustrated in an example embodiment illustrated in Fig. 2 that the catalytic bed 21 for oxidation of ammonia is formed on a surface of the solid electrolyte 20, but a catalytic bed for oxidation of ammonia may be formed even in another path where gases including ammonia flows. For example, it may be also formed on a housing of the sensor or on a surface of a third, a fourth, or a fifth electrode reactive to ammonia.

A sensor for measuring concentration of nitrogen oxides and detecting ammonia slip in accordance with the present invention may measure total concentration of nitrogen monoxide and nitrogen dioxide. Besides, it may measure discharged ammonia at the same time. In case of the sensor for measuring concentration of nitrogen oxides and detecting ammonia slip in which a nitrogen oxide sensor and an ammonia sensor share some electrodes, a solid electrolyte, a heating part, etc., its manufacturing cost is reduced. In addition, even an installation cost is saved because it is not necessary to separately install a nitrogen oxide sensor and an ammonia sensor.

Because a first electrode reactive to nitrogen oxides and a second electrode reactive to nitrogen oxides and a third electrode reactive to ammonia are formed on one side of a plate-type solid electrolyte in some example embodiments of the sensor for measuring concentration of nitrogen oxides and detecting ammonia slip in accordance with the present invention, it is possible to detect nitrogen oxides and ammonia included in gases to be measured from individual electrodes reactive to nitrogen oxides and ammonia at the same time just by exposing the one side of the solid electrolyte to the gases to be measured such as exhaust gas. Accordingly, it is possible to accurately and rapidly detect concentration of nitrogen oxides included in the gases to be measured and ammonia slip.

In some example embodiments of the sensor for measuring concentration of nitrogen oxides and detecting ammonia slip in accordance with the present invention, a fourth electrode, including a second electrode reactive to nitrogen oxide, a first electrode layer reactive to nitrogen oxide, and a third electrode layer reactive to ammonia, is formed on one side of a solid electrolyte. Furthermore, a fifth electrode, including the second electrode reactive to nitrogen oxide, a first electrode material reactive to nitrogen oxide, and a third electrode material reactive to ammonia, is formed on one side of the solid electrolyte. Accordingly, it is possible to detect nitrogen oxides and ammonia included in gases to be measured from individual electrodes reactive to the ingredients nitrogen oxides and ammonia, respectively, at the same time by exposing the one side of the solid electrolyte to the gases to be measured such as exhaust gas. Accordingly, it is possible to accurately and rapidly detect concentration of nitrogen oxides included in the gases to be measured and ammonia slip.

In some example embodiments of the sensor for measuring concentration of nitrogen oxides and detecting ammonia slip in accordance with the present invention, a first electrode reactive to nitrogen oxides and a third electrode reactive to ammonia are formed on one side of a plate-type solid electrolyte and a second electrode reactive to nitrogen oxides on the other side thereof. In addition, the second electrode reactive to nitrogen oxides is formed on the one side of a solid electrolyte and a fourth electrode, including a first electrode layer reactive to nitrogen oxides and a third electrode layer reactive to ammonia, is formed on the other side thereof. Moreover, the second electrode reactive to nitrogen oxides is formed on the one side of the solid electrolyte and a fifth electrode, including the first electrode material reactive to nitrogen oxides and the third electrode material reactive to ammonia, is formed on the other side of the solid electrolyte. For example, if the sensor for measuring concentration of nitrogen oxides and detecting ammonia slip in accordance with the present invention is installed in flowing gases to be measured, the one side of the solid electrolyte is made to face upper side of the flowing gases to be measured, and thus degradation of electrodes formed on the other side of the solid electrolyte could be controlled due to collision with the flowing gases to be measured and therefore, durability of the sensor is improved. Accordingly, concentration of nitrogen oxides and ammonia slip may be detected accurately and simultaneously through electrodes reactive to individual ingredients nitrogen oxides and ammonia over a long time of period.

Moreover, in some example embodiments of the sensor for measuring concentration of nitrogen oxides and detecting ammonia slip in accordance with the present invention, because a plate-type supporter contacting a plate-type solid electrolyte is placed on one or the other side of the solid electrolyte, it is possible to make the supporter support the solid electrolyte. Besides, if electrodes are formed on one or the other side of the solid electrolyte, the supporter is installed on the one or the other side of the solid electrolyte on which the electrodes are formed and therefore, the electrodes are placed between the solid electrolyte and the supporter. Accordingly, if the sensor for measuring concentration of nitrogen oxides and detecting ammonia slip in accordance with the present invention is installed, it is possible to prevent the gases to be measured from colliding with the electrodes and therefore, degradation of the electrodes caused by the collusion with the gases to be measured is controlled and the durability of the sensor is improved.

Besides, if a level of reactivity of an electrode formed on one side of a solid electrolyte that is reactive to gases to be measured is different from that of an electrode on the other side thereof, it is possible to control reactions of the electrodes to the gases to be measured by installing a supporter on either the one side of the solid electrolyte where the electrode is highly reactive or the other side thereof to make the electrode placed between the solid electrolyte and the supporter and adjusting amount of the gases to be measured which are contacted by the electrode. Accordingly, it is possible to adjust electrode highly reactive to the gases to be measured to set the state of detecting concentration of nitrogen oxides and ammonia slip to be optimal. For example, it is possible to adjust electrodes formed on one and the other sides of the solid electrolyte to produce the same level of reactivity.

In some example embodiments of the sensor for measuring concentration of nitrogen oxides and detecting ammonia slip in accordance with the present invention, a solid electrolyte, furthermore, is porous. Therefore, because gases to be measured pass from one side of the solid electrolyte to the other side thereof, the gases to be measured rapidly and evenly reach all electrodes formed on both sides of the solid electrolyte. Accordingly, it is possible to accurately and rapidly detect concentration of nitrogen oxides and ammonia slip.

**Claims**

1. A sensor for measuring concentration of nitrogen oxides and detecting ammonia slip, comprising:

an oxygen ion conductive solid electrolyte;

a first electrode, contacting the solid electrolyte, reactive to nitrogen oxides;

a second electrode, contacting the solid electrolyte, reactive to nitrogen oxides while being separated from the first electrode;

a third electrode, contacting the solid electrolyte, reactive to ammonia while being separated from the second electrode and being connected with the first electrode in parallel;

a power supply configured to supply power between the first electrode and the third electrode connected with each other in parallel, and the second electrode; and

a measuring instrument configured to measure electric potential difference or an electric current between the first electrode and the third electrode connected with each other in parallel, and the second electrode.

2. The sensor of Claim 1, wherein the solid electrolyte is a plate type; and the first, the second, and the third electrodes are formed on one side of the solid electrolyte.

3. The sensor of Claim 1, wherein the solid electrolyte is a plate type; the first electrode and the third electrode are formed on one side of the solid electrolyte and the second electrode is formed on the other side of the solid electrolyte.

4. The sensor of any of Claims 1 to 3, further comprising a catalytic bed for oxidation of ammonia formed on a path where ammonia flows toward the third electrode.

5. The sensor of Claim 4, wherein the catalytic bed for oxidation of ammonia is formed on a surface of the solid electrolyte.

6. The sensor of Claim 4, wherein the catalytic bed for oxidation of ammonia includes at least one of substances selected from Pt, Pd, Rh, Ir, Ru, and Ag.

7. The sensor of Claim 4, wherein the catalytic bed for oxidation of ammonia includes a porous ceramic containing precious metals scattered.

8. The sensor of Claim 4, wherein the catalytic bed for oxidation of ammonia includes at least one of substances selected from $CO_3O_4$, $MnO_2$, $V_2O_5$, $Ni-Al_2O_3$, $Fe-Al_2O_3$, $Mn-Al_2O_3$, $CuO-Al_2O_3$, $Fe_2O_3-Al_2O_3$, $Fe_2O_3-TiO_2$, and $Fe_2O_3-ZrO_2$.

9. The sensor of Claim 4, wherein the catalytic bed for oxidation of ammonia includes ion exchanging zeolite.

10. The sensor of any of Claims 1 to 9, wherein the third electrode includes at least one of substances selected from ZnO, $SnO_2$, and $In_2O_3$.

11. A sensor for measuring concentration of nitrogen oxides and detecting ammonia slip, comprising:

an oxygen ion conductive solid electrolyte;

a second electrode, contacting the solid electrolyte, reactive to nitrogen oxides;

a fourth electrode including a first electrode layer, contacting the solid electrolyte, reactive to nitrogen oxides while being separated from the second electrode, and a third electrode layer reactive to ammonia; and

a power supply configured to supply power between the second electrode and the fourth electrode; and

a measuring instrument configured to measure electric potential difference or an electric current between the second electrode and the fourth electrode.

12. The sensor of Claim 11, wherein the solid electrolyte is a plate type; and the second electrode and the fourth electrode are formed on one side of the solid electrolyte.

13. The sensor of Claim 11, wherein the solid electrolyte is a plate type; and the second electrode is formed on one side of the solid electrolyte and the fourth electrode is formed on the other side thereof.

14. The sensor of any of Claims 11 to 13, further comprising a catalytic bed for oxidation of ammonia formed on a path where ammonia flows toward the fourth electrode.

15. The sensor of Claim 14, wherein the catalytic bed for oxidation of ammonia is formed on a surface of the solid electrolyte.

16. A sensor for measuring concentration of nitrogen oxides and detecting ammonia slip, comprising:

an oxygen ion conductive solid electrolyte;
a second electrode, contacting the solid electrolyte, reactive to nitrogen oxides;
a fifth electrode including a first electrode material, contacting the solid electrolyte, reactive to nitrogen oxides while being separated from the second electrode, and the third electrode material reactive to ammonia;
a power supply configured to supply power between the second electrode and the fifth electrode; and
a measuring instrument configure to measure electric potential difference or an electric current between the second electrode and the fifth electrode.

17. The sensor of Claim 16, wherein the solid electrolyte is a plate type; and the second electrode and the fifth electrode are formed on one side of the solid electrolyte.

18. The sensor of Claim 16, wherein the solid electrolyte is a plate type; and the second electrode is formed on one side of the solid electrolyte and the fifth electrode is formed on the other side of the solid electrolyte.

19. The sensor of any of Claims 16 to 18, further comprising a catalytic bed for oxidation of ammonia formed on a path where ammonia flows towards the fifth electrode.

20. The sensor of Claim 19, wherein the catalytic bed for oxidation of ammonia is formed on a surface of the solid electrolyte.

21. The sensor of any of Claims 1 to 20, further comprising a plate-type supporter contacting the solid electrolyte; wherein the solid electrolyte is a plate type; and the supporter is placed on one or the other side of the solid electrolyte.

22. The sensor of any of Claims 1 to 21, wherein the solid electrolyte is porous.

FIG.1

FIG.2

## FIG.3

## FIG.4

NITROGEN OXIDE | NITROGEN OXIDE AND AMMONIA 250PPM | NITROGEN OXIDE AND AMMONIA 500PPM

## FIG.5

## FIG.6

## FIG.7

FIG.8

FIG.9

FIG.10

FIG.11

## INTERNATIONAL SEARCH REPORT

| International application No. |
| --- |
| **PCT/KR2017/003461** |

| | |
| --- | --- |
| A. CLASSIFICATION OF SUBJECT MATTER | |
| *G01N 27/327(2006.01)i, G01N 33/00(2006.01)i, G01N 25/32(2006.01)i* | |
| According to International Patent Classification (IPC) or to both national classification and IPC | |

**B. FIELDS SEARCHED**

Minimum documentation searched (classification system followed by classification symbols)
G01N 27/327; G01F 1/64; G01N 27/26; G01N 35/00; G01N 27/407; G01N 33/00; G01N 25/32

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: nitrogen oxide, ammonia, slip, sensor, gas and detection

**C. DOCUMENTS CONSIDERED TO BE RELEVANT**

| Category* | Citation of document, with indication, where appropriate, of the relevant passages | Relevant to claim No. |
| --- | --- | --- |
| Y | KR 10-2008-0075104 A (DELPHI TECHNOLOGIES, INC.) 14 August 2008<br>See paragraphs [0015]-[0033], claim 5 and figure 1. | 1-22 |
| Y | US 2010-0077833 A1 (WANG et al.) 01 April 2010<br>See paragraphs [0007]-[0017], claim 1 and figure 1. | 1-22 |
| A | US 2003-0062264 A1 (KITANOYA et al.) 03 April 2003<br>See claim 1 and figure 1. | 1-22 |
| A | US 2004-0118703 A1 (WANG et al.) 24 June 2004<br>See figure 1. | 1-22 |
| A | US 4985126 A (HAEFELE et al.) 15 January 1991<br>See figure 4. | 1-22 |

☐ Further documents are listed in the continuation of Box C.　　☒ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 24 JULY 2017 (24.07.2017) | **24 JULY 2017 (24.07.2017)** |

| Name and mailing address of the ISA/KR | Authorized officer |
| --- | --- |
| Korean Intellectual Property Office<br>Government Complex-Daejeon, 189 Seonsa-ro, Daejeon 302-701,<br>Republic of Korea | |
| Facsimile No. +82-42-481-8578 | Telephone No. |

Form PCT/ISA/210 (second sheet) (January 2015)

INTERNATIONAL SEARCH REPORT
Information on patent family members

International application No.

**PCT/KR2017/003461**

| Patent document cited in search report | Publication date | Patent family member | Publication date |
|---|---|---|---|
| KR 10-2008-0075104 A | 14/08/2008 | EP 1772599 A2 | 11/04/2007 |
| | | EP 1772599 A3 | 04/03/2009 |
| | | EP 1772599 B1 | 02/03/2011 |
| | | EP 1943484 A2 | 16/07/2008 |
| | | EP 1943507 A1 | 16/07/2008 |
| | | EP 1943507 B1 | 18/09/2013 |
| | | JP 2009-511859 A | 19/03/2009 |
| | | JP 5022371 B2 | 12/09/2012 |
| | | KR 10-1275972 B1 | 14/06/2013 |
| | | US 2007-0079597 A1 | 12/04/2007 |
| | | US 2007-0080074 A1 | 12/04/2007 |
| | | US 2007-0080075 A1 | 12/04/2007 |
| | | US 7294252 B2 | 13/11/2007 |
| | | US 7757478 B2 | 20/07/2010 |
| | | WO 2007-044302 A2 | 19/04/2007 |
| | | WO 2007-044302 A3 | 07/05/2009 |
| | | WO 2007-044387 A1 | 19/04/2007 |
| US 2010-0077833 A1 | 01/04/2010 | EP 2169395 A2 | 31/03/2010 |
| | | EP 2169395 A3 | 02/03/2011 |
| | | US 2010-0161242 A1 | 24/06/2010 |
| | | US 8051700 B2 | 08/11/2011 |
| | | US 8103458 B2 | 24/01/2012 |
| US 2003-0062264 A1 | 03/04/2003 | EP 1293776 A2 | 19/03/2003 |
| | | EP 1293776 A3 | 11/02/2004 |
| | | JP 2003-083933 A | 19/03/2003 |
| US 2004-0118703 A1 | 24/06/2004 | US 2006-0266659 A1 | 30/11/2006 |
| | | US 2010-0282618 A1 | 11/11/2010 |
| | | US 2010-0282619 A1 | 11/11/2010 |
| | | US 7074319 B2 | 11/07/2006 |
| | | US 7828955 B2 | 09/11/2010 |
| | | US 8257575 B2 | 04/09/2012 |
| | | US 8257576 B2 | 04/09/2012 |
| US 4985126 A | 15/01/1991 | DE 000003610363 A1 | 01/10/1987 |
| | | EP 0241751 A2 | 21/10/1987 |
| | | EP 0241751 B1 | 18/11/1993 |
| | | EP 0241751 B2 | 07/11/2001 |
| | | JP 2582255 B2 | 19/02/1997 |
| | | JP 63-006454 A | 12/01/1988 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 100864381